# EUROPÄISCHE PATENTSCHRIFT

(11) **EP 0 353 526 B1**
(45) Veröffentlichungstag und Bekanntmachung des Hinweises auf die Patenterteilung: **26.05.1993**
(21) Anmeldenummer: 89113012.2
(22) Anmeldetag: 15.07.1989
(51) Int. Cl.: A61K 31/53, C07D 253/06

(54) **Triazindion-Derivate enthaltendes Mittel gegen Fischparasiten**
Agent against fish parasites containing triazinedione derivatives
Agent renfermant des dérivés de la triazindione contre les parasites

(30) Priorität: 30.07.1988 DE 3826058
(43) Veröffentlichungstag der Anmeldung: 07.02.1990
(73) Patentinhaber: BAYER AG, 51368 Leverkusen (DE)
(72) Erfinder: Mehlhorn, Heinz, Prof. Dr., D-4040 Neuss-Üdesheim (DE); Schmahl, Günter, Dr., D-4630 Bochum (DE); Lindner, Werner, Dr., D-5000 Koeln 80 (DE); Haberkorn, Axel, Prof. Dr., D-5600 Wuppertal 1 (DE)

(56) Entgegenhaltungen:
- EP-A- 0 170 316
- EP-A- 0 232 932
- EP-A- 0 330 041
- WO-A-86/00072
- US-A- 3 912 723

## Beschreibung

Die vorliegende Erfindung betrifft Mittel, die 1,2,4-Triazindione enthalten gegen Fischparasiten, insbesondere parasitäre Protozoen (Einzeller) und Metazoen (Vielzeller).

Zu den Protozoen und Metazoen gehören Klassen, die als Parasiten bei Fischen weit verbreitet sind. Bei der Massentierhaltung in großen Zuchtanlagen stellen sie ein ernstes Problem dar, da sich ein Befall rasch über den ganzen Bestand ausbreiten kann. Vor allem bei jungen und empfindlichen Fischen stellen diese Parasiten ein großes Problem für die Aufzucht dar und führen zur erheblichen Verlusten.

Parasitäre Protozoen und Metazoen heften sich zum Teil auf Haut und Kiemen der Fische und verursachen dadurch Verletzungen der Haut, durch die die Fische anfällig für Infektionen durch Bakterien, Viren oder Pilze werden. Sie sind außerdem Vektoren für Virusinfektionen. Parasitäre Protozoen und Metazoen befallen zum Teil auch innere Organe der Fische (z.B. Darm, Knochen) und führen zu Verwachsungen oder zum Tod der Fische.

Nur wenige Mittel zur Bekämpfung der parasitären Protozoen und Metazoen sind bekannt. Diese wirken jedoch nicht immer voll befriedigend. Außerdem besitzen sie meist nur ein enges Wirkungsspektrum gegen bestimmte Parasiten. Gegen andere Parasiten, z.B. Myxozoen oder Microsporidien fehlen wirksame Mittel völlig.

Es wurde gefunden, daß die substituierten 1,2,4-Triazindione der allgemeinen Formel (I) 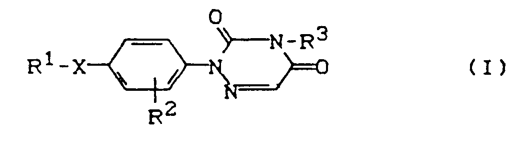
in welcher
- R¹: für aromatische oder über Kohlenstoff gebundene heteroaromatische Reste steht, die gegebenenfalls substituiert sind
- X: für 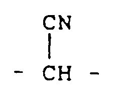 steht,
- R²: für einen oder mehrere, gleiche oder verschiedene Reste der Gruppe Wasserstoff, Halogen, Nitro, Alkyl, Alkoxy, Halogenalkyl, Halogenalkoxy
- R³: für Wasserstoff, gegebenenfalls substituiertes Alkyl, Alkenyl, Alkinyl, Aralkyl steht
sowie ihre Salze mit Basen zur Bekämpfung von Fischparasiten, insbesondere parasitäre Protozoen und Metazoen, z.B. Plathelminthen, verwendet werden können.

Die Triazindione sind z. T. bekannt aus EP-OS 170 316 bzw. sind Gegenstand der älteren nicht vorveröffentlichten Anmeldung der Anmelderin EP-OS 330 041.

Bevorzugt verwendet werden Verbindungen der Formel (I), in welcher
- R¹: für gegebenenfalls durch Halogen, Alkyl, Cyano, Nitro, O-Alkyl, S-Alkyl, Halogenalkyl, substituiertes Phenyl, Thiazolyl, Oxazolyl, Benzthiazolyl oder Benzoxazolyl steht.
- X: für 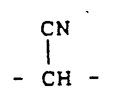 steht
- R²: für Halogen oder C₁₋₆-Alkyl steht
- R³: für Wasserstoff oder C₁-C₄-Alkyl, insbesondere Methyl, steht.

Besonders bevorzugt werden Verbindungen der Formel (I) verwendet, in welcher
- X: für 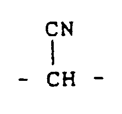 steht,
- R¹: für Thiazolyl, Benzthiazolyl, Benzoxazolyl oder Phenyl,
die gegebenenfalls durch C₁₋₄-Alkyl, insbesondere Methyl, C₁₋₄-Halogenalkyl insbesondere Trifluormethyl, Halogen, insbesondere Chlor, Brom, Fluor, Nitro, CN, C₁₋₄-Alkoxy insbesondere Methoxy, C₁₋₄-Halogenalkoxy insbesondere Trifluormethoxy, C₁₋₄-Alkylthio insbesondere Methylthio, C₁₋₄-Halogenalkylthio insbesondere Trifluormethylthio substituiert sind, steht,
- R²: für einen oder mehrere Reste der Gruppe Wasserstoff oder Halogen insbesondere Chlor, Brom, C₁₋₄-Alkyl insbesondere Methyl steht,
- R³: für Wasserstoff steht.

Besonders bevorzugt werden auch Verbindungen der Formel (I) verwendet, in welcher
- X: für 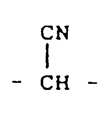 steht
- R¹: für gegebenenfalls durch Chlor, Methyl, Trifluormethyl substituiertes Phenyl steht,
- R²: für einen oder mehrere gleiche oder verschiedene Reste aus der Gruppe Wasserstoff, Chlor, Methyl steht,
- R³: für Wasserstoff oder Methyl steht.

Insbesondere seien genannt:
2-Chlor-α-(4-chlorphenyl)-4-(4,5-dihydro-3,5-dioxo-1,2,4-triazin-2(3H)-yl)-phenylacetonitril (Clazuril) und
2,6-Dichlor-α-(4-chlorphenyl)-4-(4,5-dihydro-3,5-dioxo-1,2,4-triazin-2(3H)-yl)-phenylacetonitril (Diclazuril).

Es war bekannt, daß sich 1,2,4-Triazindione der Formel (I), in welcher
- X: für 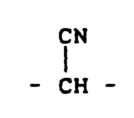 und
- R¹: für Phenyl steht,
zur Bekämpfung von Coccidien der Säugetiere und des Geflügels einsetzen lassen. Auch für die noch nicht bekannten Verbindungen der Formel (I) wird diese Wirkung angegeben. Es war nichts darüber bekannt, daß die Verbindungen der Formel (I) eingesetzt werden können, um Parasiten bei Fischen zu bekämpfen.

Zu den Parasiten bei Fischen gehören aus dem Unterreich der Protozoen Spezies des Stammes der Ciliata, z.B. Ichthyophthirius multifiliis, Chilodonella cyprini, Trichodina spp., Glossatella spp., Epistylis spp. des Stammes der Myxosporidia, z.B. Myxosoma cerebralis, Myxidium spp., Myxobolus spp., Heneguya spp., Hoferellus spp., der Klasse der Mikrosporidia z.B. Glugea spp., Thelohania spp., Pleistophora spp., aus dem Stamm der Plathelminthen: Trematoden; Monogenea z.B. Dactylogyrus spp., Gyrodactylus ssp., Pseudodactylogyrus spp., Diplozoon spp., Cestoden, z.B. aus den Gruppen der Caryphyllidea (z.B. Caryophyllaeus laticeps), Pseudophyllidea (z.B. Diphyllobothrium spp.), Tetraphyllidea (z.B. Phyllobothrium spp.) und Protocepthalida (z.B. Arten der Gattung Proteocephalus) und aus dem Stamm der Arthropoda verschiedene parasitische Gustaceen, insbesondere aus den Unterklassen der Branchiura (Fischläuse) und Copepoda (Ruderfußkrebse) sowie den Ordnungen der Isopoda (Asseln) und Amphipoda (Flohkrebse).

Zu den Fischen gehören Nutz-, Zucht-, Aquarien- und Zierfische aller Altersstufen, die in Süß- und Salzwasser leben. Zu den Nutz- und Zuchtfischen zählen z.B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z.B. Plagioscion, Channel catfish. Besonders geeignet sind die erfindungsgemäßen Mittel zur Behandlung von Fischbrut z.B. Karpfen von 2-4 cm Körperlänge. Sehr gut geeignet sind die Mittel auch in der Aalmast.

Die Behandlung der Fische erfolgt entweder oral, z.B. über das Futter oder durch Kurzzeitbehandlung, "medizinisches Bad", in das die Fische eingesetzt und in dem sie eine Zeitlang (Minuten bis mehrere Stunden) z.B. beim Umsetzen von einem Zuchtbecken zum anderen gehalten werden.

Es kann aber auch eine vorübergehende oder dauernde Behandlung des Lebensraums der Fisch (z.B. ganzer Teichanlagen, Aquarien, Tanks oder Becken), in denen die Fische gehalten werden, erfolgen.

Der Wirkstoff wird in Zubereitungen verabreicht, die den Anwendungen angepaßt sind.

Zubereitungen zur oralen Anwendungen sind Pulver, Granulate, Lösungen, Emulsions- oder Suspensionskonzentrate die als Futterzusätze mit dem Futter homogen vermischt werden.

Die Zubereitungen werden in an sich bekannter Weise hergestellt, indem man den Wirkstoff mit festen oder flüssigen Trägerstoffen gegebenenfalls unter Zusatz weiterer Wirkstoffe sowie Emulgier- oder Dispergiermittel, Lösungsvermittler, Farbstoffe, Antioxidantien, Konservierungsstoffe vermischt.

Zu den festen Trägerstoffen zählen z.B. natürliche Gesteinsmehle wie Kaoline, Tonerden, Talkum, Kreide, Diatomeenerde, organische Trägerstoffe wie Zucker, Rohr-, Milch-, Traubenzucker, Getreideprodukte wie Getreidemehle oder -schrote, Stärke, Tiermehle, Cellulose, Milchpulver, anorganische Trägerstoffe wie Kochsalz, Carbonate wie Calciumcarbonat, Hydrogencarbonate, Aluminiumoxide, Kieselsäure, Silikate.

Zu den flüssigen Trägerstoffen und Lösungsvermittlern zählen:
Wasser, Alkanole wie Ethanol, Isopropanol, Glykole wie Ethylenglykol, Propylenglykol, Polyethylenglykole, Polypropylenglykole und ihre Copolymer, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmonobutylether, Ketone wie Aceton, Methylethylketon, aromatische und/oder aliphatische Kohlenwasserstoffe, pflanzliche oder synthetische Öle, DMF, DMSO, Dimethylacetamid, N-Methylpyrrolidon, 2-Dimethyl-4-oxymethylen-1,3-dioxalon.

Zu den Dispergier- und Emulgiermitteln zählen:
nichtionogene Tenside wie polyoxyethyliertes Rizinusöl, polyoxyethyliertes Sorbitan-monooleat, Sorbitan-monostearat, Glycerinmonostearat, Polyoxyethylenstearat, Alkylphenolpolyglykolether, ampholytische Tenside wie Di-Na-N-lauryl-β-iminodipropionat oder Lecithin, anionaktive Tenside wie Na-Laurylsulfat, Fettalkoholethersulfate, Mono/Dialkylpolyglykoletherorthophosphorsäureester-monoethanolaminsalze, kationaktive Tenside wie Cetyltrimethylammoniumchlorid.

Die Konzentration des Wirkstoffs, liegt in den Zubereitungen bei 1 ppm bis 10 Gew.-%.

Bevorzugte Zubereitungen zur Kurzzeitbehandlung in der Anwendung als "medizinisches Bad" z.B. bei der Behandlung beim Umsetzen der Fische oder zur Behandlung des Lebensraums (Teichbehandlung) der Fische sind Lösungen des Wirkstoffs in einem oder mehreren polaren Lösungsmitteln, die bei Verdünnen mit Wasser alkalisch reagieren.

Zur Herstellung dieser Lösungen wird der Wirkstoff in einem polaren, wasserlöslichen Lösungsmittel gelöst, welches entweder alkalisch reagiert oder dem eine alkalische wasserlösliche Substanz zugefügt wird. Letztere wird vorteilhaft ebenfalls im Lösungsmittel gelöst, kann aber auch in dem Lösungsmittel suspendiert sein und sich erst im Wasser lösen. Dabei soll das Wasser nach Zusatz der Wirkstofflösung einen pH-Wert von 7-10, vorzugsweise aber einen pH-Wert von 8-10 haben.

Die Konzentration des Wirkstoffes kann im Bereich von 0,5-50 % liegen, vorzugsweise aber in einem Bereich von 1-25 %.

Als Lösungsmittel kommen alle wasserlöslichen Lösungsmittel in Betracht, in denen der Wirkstoff in genügender Konzentration löslich ist und die physiologisch unbedenklich sind.

Dies sind Ethylalkohol, Isopropylalkohol, Benzylalkohol, Glycerin, Propylenglykol, Polyethylenglykole, Poly(oxoethylen)-poly(oxypropylen)-Polymere, basische Alkohole wie Mono-, Di- und Triethanolamin, Ketone wie Aceton oder Methylethylketon, Ester wie Milchsäureethylester ferner N-Methylpyrrolidon, Dimethylacetamid, Dimethylformamid, ferner Dispergier- und Emulgiermittel wie polyoxyethyliertes Rizinusöl, Polyethylenglykol-Sorbitan-Monooleat, Polyethylenglykolstearat, oder Polyethylenglykolether, Polyethylenglykol-Alkylamine.

Als Basen zur Einstellung des alkalischen pH-Wertes seien genannt organische Basen wie basische Aminosäuren wie L- bzw. D,L-Arginin, L- bzw. D, L-Lysin, Methylglucosamin, Glucosamin, 2-Amino-2-hydroxymethylpropandiol-(1,3) ferner wie N,N,N',N'-tetrakis-(2-hydroxypropyl)-ethylendiamin oder Polyether-Tetrol auf der Basis Ethylendiamin (M.G. 480-420), anorganische Basen, wie Ammoniak oder Natriumcarbonat-gegebenenfalls unter Zugabe von Wasser.

Die Zubereitungen können auch 0,1 bis 20 Gew.-%, vorzugsweise 0,1-10 Gew.-% anderer Formulierhilfsstoffe, wie Antioxidantien, Tenside, Suspensionsstabilisatoren und Verdickungsmittel wie z.B. Methylcellulose, Alginate, Polysaccharide, Galaktomannane und kolloidale Kieselsäure enthalten. Der Zusatz von Farbe, Aroma und Aufbaustoffen zur Tierernährung ist ebenfalls möglich. Auch Säuren, die mit der vorgelegten Base zusammen ein Puffersystem bilden oder den pH der Lösung reduzieren, sind hier zu nennen.

Die Konzentration des Wirkstoffs bei der Anwendung hängt ab von Art und Dauer der Behandlung, sowie Alter und Zustand der behandelten Fische. Sie beträgt z.B. bei Kurzzeitbehandlung 2-50 mg Wirkstoff pro Liter Wasser bevorzugt 5-10 mg pro Liter, bei einer Behandlungsdauer von 3-4 Stunden. Bei der Behandlung von jungen Karpfen wird z.B. mit einer Konzentration von 5-10 mg/l und einer Behandlungsdauer von ca. 1-4 Stunden gearbeitet.

Aale werden mit Konzentrationen von ca. 5 mg/l ca. 4 Stunden behandelt.

Bei längerer Behandlungsdauer oder bei Dauerbehandlung kann die Konzentration entsprechend niedriger gewählt werden.

Bei Teichbehandlungen können 0,1-5 mg Wirkstoff pro Liter Wasser verwendet werden.

Zubereitungen zur Anwendung als Futterzusatz sind z.B. wie folgt zusammengesetzt:

| | | |
|---|---|---|
| a) | Wirkstoff der Formel I | 1 - 10 Gewichtsteile |
| | Sojabohnen-Protein | 49 - 90 Gewichtsteile |
| b) | Wirkstoff der Formel I | 0,5 - 10 Gewichtsteile |
| | Benzylalkohol | 0,08 - 1,4 Gewichtsteile |
| | Hydroxypropylmethylcellulose | 0 - 3,5 Gewichtsteile |
| | Wasser | Rest ad 100 |

Zubereitungen zur Anwendung bei "medizinischen Bädern" und zur Teichbehandlung sind z.B. wie folgt zusammengesetzt und hergestellt.

| | | |
|---|---|---|
| c) | Wirkstoff gemäß Formel I | 5,0 g |
| | Propylenglykol | 50,0 g |
| | Natriumcarbonat | 5,0 g |
| | Wasser | ad 100 ml |
| d) | Wirkstoff gemäß Formel I | 5,0 g |
| | Monoethanolamin | 10 g |
| | N-Methylpyrrolidon | ad 100 ml |
| e) | Wirkstoff gemäß Formel I | 2,5 g |
| | Natriumcarbonat | 5,0 g |
| | Polyethylenglykol200 | ad 100 ml |

Der Wirkstoff wird unter Erwärmen im Polyethylenglykol gelöst und Natriumcarbonat darin suspendiert.

## Patentansprüche

1. Verwendung von substituierten 1,2,4-Triazindionen der allgemeinen Formel (I) 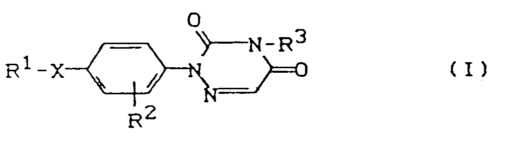 in welcher
R¹ für aromatische oder über Kohlenstoff gebundene heteroaromatische Reste steht, die gegebenenfalls substituiert sind
X für 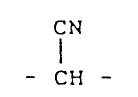 steht,
R² für einen oder mehrere, gleiche oder verschiedene Reste der Gruppe Wasserstoff, Halogen, Nitro, Alkyl, Alkoxy, Halogenalkyl, Halogenalkoxy
R³ für Wasserstoff, gegebenenfalls substituiertes Alkyl, Alkenyl, Alkinyl, Aralkyl steht
sowie ihrer Salze mit Basen zur Herstellung von Arzneimitteln zur Bekämpfung von Fischparasiten.

2. Verwendung gemäß Anspruch 1 von Verbindungen der Formel (I), in welcher
R¹ für gegebenenfalls durch Halogen, Alkyl, Cyano, Nitro, O-Alkyl, S-Alkyl, Halogenalkyl, substituiertes Phenyl, Thiazolyl, Oxazolyl, Benzthiazolyl oder Benzoxazolyl steht.
X für 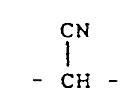 steht
R² für Halogen oder C₁₋₆-Alkyl steht
R³ für Wasserstoff oder C₁-C₄-Alkyl, insbesondere Methyl, steht.

3. Verwendung gemäß Anspruch 1 von Verbindungen der Formel (I), in welcher
X für 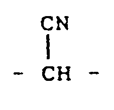 steht,
R¹ für Thiazolyl, Benzthiazolyl, Benzoxazolyl oder Phenyl,
die gegebenenfalls durch C₁₋₄-Alkyl, insbesondere Methyl, C₁₋₄-Halogenalkyl insbesondere Trifluormethyl, Halogen, insbesondere Chlor, Brom, Fluor, Nitro, CN, C₁₋₄-Alkoxy insbesondere Methoxy, C₁₋₄-Halogenalkoxy insbesondere Trifluormethoxy, C₁₋₄-Alkylthio insbesondere Methylthio, C₁₋₄-Halogenalkylthio insbesondere Trifluormethylthio substituiert sind, steht,
R² für einen oder mehrere Reste der Gruppe Wasserstoff oder Halogen insbesondere Chlor, Brom, C₁₋₄-Alkyl insbesondere Methyl steht,
R³ für Wasserstoff steht.

4. Verwendung gemäß Anspruch 1 von Verbindungen der Formel (I), in welcher
X für 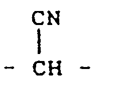 steht
R¹ für gegebenenfalls durch Chlor, Methyl, Trifluormethyl substituiertes Phenyl steht,
R² für einen oder mehrere gleiche oder verschiedene Reste aus der Gruppe Wasserstoff, Chlor, Methyl steht,
R³ für Wasserstoff oder Methyl steht.

5. Verwendung gemäß Anspruch 1 von
2-Chlor-α-(4-chlorphenyl)-4-(4,5-dihydro-3,5-dioxo-1,2,4-triazin-2(3H)-yl)-phenylacetonitril (Clazuril) und
2,6-Dichlor-α-(4-chlorphenyl)-4-(4,5-dihydro-3,5-dioxo-1,2,4-triazin-2(3H)-yl)-phenylacetonitril (Diclazuril).

## Claims

1. Use of substituted 1,2,4-triazinediones of the general formula (I) 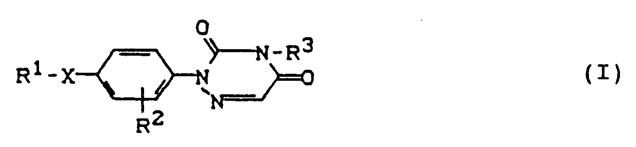 in which
R¹ represents optionally substituted aromatic radicals, or represents optionally substituted heteroaromatic radicals which are bonded via carbon,
X represents 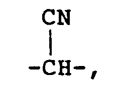
R² represents one or more identical or different radicals from the group comprising hydrogen, halogen, nitro, alkyl, alkoxy, halogenoalkyl, halogenoalkoxy,
R³ represents hydrogen, optionally substituted alkyl, alkenyl, alkinyl, aralkyl,
and of their salts with bases for the preparation of medicaments for combating fish parasites.

2. Use according to Claim 1 of compounds of the formula (I) in which
R¹ represents phenyl, thiazolyl, oxazolyl, benzothiazolyl, or benzoxazolyl, each of which is optionally substituted by halogen, alkyl, cyano, nitro, O-alkyl, S-alkyl or halogenoalkyl,
X represents 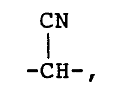
R² represents halogen or C₁₋₆-alkyl, and
R³ represents hydrogen or C₁-C₄-alkyl, in particular methyl.

3. Use according to Claim 1 of compounds of the formula (I) in which
X represents 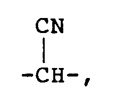
R¹ represents thiazolyl, benzothiazolyl, benzoxazolyl or phenyl,
each of which is optionally substituted by C₁₋₄-alkyl, in particular methyl, C₁₋₄-halogenoalkyl, in particular trifluoromethyl, halogen, in particular chlorine, bromine, fluorine, nitro, CN, C₁₋₄-alkoxy, in particular methoxy, C₁₋₄-halogenoalkoxy, in particular trifluoromethoxy, C₁₋₄-alkylthio, in particular methylthio, C₁₋₄-halogenoalkylthio, in particular trifluoromethylthio,
R² represents one or more radicals from the group comprising hydrogen and halogen, in particular chlorine, bromine, C₁₋₄-alkyl, in particular methyl,
R³ represents hydrogen.

4. Use according to Claim 1 of compounds of the formula (I), in which
X represents 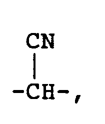
R¹ represents phenyl which is optionally substituted by chlorine, methyl or trifluoromethyl,
R² represents one or more identical or different radicals from the group comprising hydrogen, chlorine and methyl, and
R³ represents hydrogen or methyl.

5. Use according to Claim 1 of
2-chloro-α-(4-chlorophenyl)-4-(4,5-dihydro-3,5-dioxo-1,2,4-triazine-2(3H)-yl)-phenylacetonitrile (clazuril) and
2,6-dichloro-α-(4-chlorophenyl)-4-(4,5-dihydro-3,5-dioxo-1,2,4-triazine-2(3H)-yl)-phenylacetonitrile (diclazuril).

## Revendications

1. Utilisation de 1,2,4-triazinediones substituées de formule générale (I) 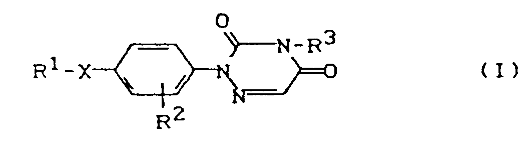 dans laquelle
R¹ représente des restes aromatiques ou hétéroaromatiques liés par l'intermédiaire de carbone, qui sont éventuellement substitués,
X représente 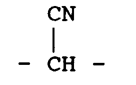
R² représente un ou plusieurs restes, identiques ou différents, du groupe hydrogène, halogéno, nitro, alkyle, alkoxy, halogénalkyle, halogénalkoxy,
R³ représente l'hydrogène, un groupe alkyle éventuellement substitué, alcényle, alcynyle, aralkyle,
ainsi que leurs sels avec des bases pour la préparation de médicaments destinés à combattre des parasites de poissons.

2. Utilisation suivant la revendication 1 de composés de formule (I), dans laquelle
R¹ est un groupe phényle, thiazolyle, oxazolyle, benzothiazolyle ou benzoxazolyle portant éventuellement un substituant halogéno, alkyle, cyano, nitro, O-alkyle, S-alkyle, halogénalkyle,
X représente 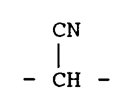
R² est un halogène ou un groupe alkyle en C₁ à C₆,
R³ est l'hydrogène ou un groupe alkyle en C₁ à C₄, notamment un groupe méthyle.

3. Utilisation suivant la revendication 1 de composés de formule (I), dans laquelle
X représente 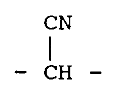
R¹ est un groupe thiazolyle, benzothiazolyle, benzoxazolyle ou phényle,
chacun de ces groupes étant éventuellement substitué par un radical alkyle en C₁ à C₄, notamment méthyle, halogénalkyle en C₁ à C₄, notamment trifluorométhyle, halogéno, notamment chloro, bromo, fluoro, nitro, CN, alkoxy en C₁ à C₄, notamment méthoxy, halogénalkoxy en C₁ à C₄, notamment trifluorométhoxy, alkylthio en C₁ à C₄, notamment méthylthio, halogénalkylthio en C₁ à C₄, notamment trifluorométhylthio,
R² représente un ou plusieurs restes du groupe de l'hydrogène ou d'un halogène, notamment chlore, brome, alkyle en C₁ à C₄, notamment méthyle,
R³ est l'hydrogène.

4. Utilisation suivant la revendication 1 de composés de formule (I), dans laquelle
X représente 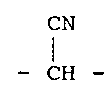
R¹ est un groupe phényle éventuellement substitué par un radical chloro, méthyle, trifluorométhyle,
R² représente un ou plusieurs restes identiques ou différents du groupe hydrogène, chloro, méthyle,
R³ est l'hydrogène ou un groupe méthyle.

5. Utilisation suivant la revendication 1 du 2-chloro-α-(4-chlorophényl)-4-(4,5-dihydro-3,5-dioxo-1,2,4-triazine-2(3H)-yl)phénylacétonitrile (clazuril) et
du 2,6-dichloro-α-(4-chlorophényl)-4-(4,5-dihydro-3,5-dioxo-1,2,4-triazine-2(3H)-yl)phénylacétonitrile (diclazuril).
